# EUROPEAN PATENT APPLICATION

(11) **EP 3 620 462 A1**
(43) Date of publication of application: **11.03.2020**
(21) Application number: 18192529.8
(22) Date of filing: 04.09.2018
(51) Int. Cl.: C07K 7/56

(54) **PROCESS FOR THE PREPARATION OF CASPOFUNGIN**

(71) Applicant: Xellia Pharmaceuticals ApS, 2300 Copenhagen S (DK)
(72) Inventor: GUNNES, Sølvi, N-0380 Oslo (NO)
(74) Representative: Onsagers AS

(57) **Abstract**

The present invention provides a process for the production of a compound of formula I

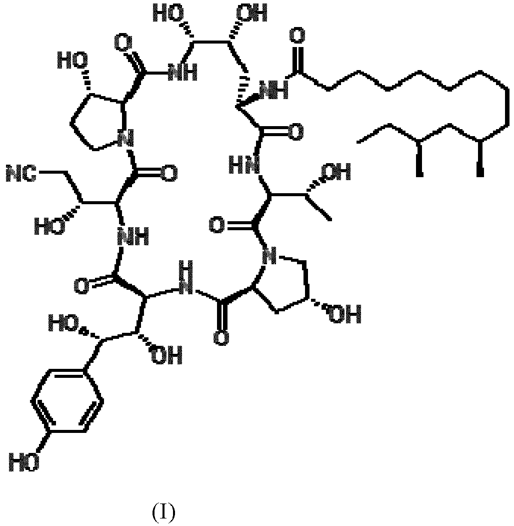

## Description

### Field of the invention

The present invention relates to an improved process for the preparation of echinocandin derivatives, in particular caspofungin represented by formula III:

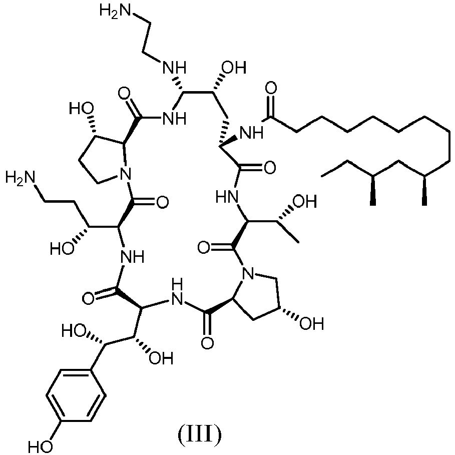

### Background

Caspofungin is a semi-synthetic antifungal agent belonging to the class of echinocandins. The drug is prepared by synthetic derivatisation of pneumocandin B₀ which is obtained by fermentation of the fungus *Glarea lozoyensis.* Caspofungin inhibits the biosynthesis of β-(1,3)-D-glucan, an integral component of the fungal cell wall, and it is indicated for the treatment of invasive aspergillosis in patients who are refractory to or intolerant of other therapies, as well as empirical therapy for presumed fungal infections in febrile, neutropenic patients. Caspofungin is marketed as its diacetate salt by Merck & Co., under the trade name Cancidas®.

Caspofungin as a compound is claimed in the patent US 5,378,804 issued to Merck & Co. The drug was prepared in a lengthy synthetic sequence in 0.7% overall yield from pneumocandin B₀. The first two synthetic steps were the subject of the patent application EP 0 535 967 A2. Reduction of the primary amide was effected in two steps, *i.e.* dehydration with cyanuric chloride affording an intermediate nitrile, in 29-56% yield, which was reduced with sodium borohydride in the presence of cobalt(II) chloride. The aminal side-chain was introduced *via* substitution of 2-aminoethanethiol for the hemiaminal hydroxy function, oxidation to the sulfone, followed by substitution with 1,2-diaminoethane.

The pneumocandin Bo reduction product, compound of formula III in Scheme 1, is claimed as a compound in the patent US 5,939,384.

Journet and co-workers describe an improved two-step reduction of the primary amide in an echinocandin analogue (Journet et al., J. Org. Chem. 1999, 64, 2411-2417). When the cyanuric chloride dehydration was performed at -30 °C with careful control of the water content an efficient reaction was obtained. However, about 4% of an inseparable benzyl isomer was also formed. The resulting nitrile function was later reduced under catalytic hydrogenation conditions in high yield.

It is noted that the cyanuric chloride dehydration is very sensitive to the amount of water present in the reaction, as well as the temperature. The amount of water must be within a carefully controlled range to obtain an efficient reaction. The fact that pneumocandin B₀ is very hygroscopic contributes to difficulties in obtaining a reproducible reaction, in particular when the reaction is run on a large scale. This class of lipopeptides have poor solubility in organic solvents and have micellar and soap like behaviour and also displays poor crystallinity. It is further noted that pneumocandin B₀ is highly unstable to both acidic and basic conditions because of lability imparted by the hemiaminal. (Buffard et al. Tet. Lett. 1995, Vol 36, No 9. 1405-1408.). Accelerated ionization or ring opening of pneumocandin B₀ in water at the hemiaminal has been reported when pH is outside 5 to 8. Also anhydrous degradation when pH<5 has been reported (Singh et al., Nat. Prod. Rep. 2014, 31, 15-34).

Reduction of the primary amide with borane complexes in one step directly to the corresponding amine is disclosed in US 5,552,521. When pneumocandin B₀ was treated with an excess of borane-dimethyl sulfide complex in dry THF at 0 °C the reduction product was obtained in 43% yield. The process was improved further when 2-aminoethanethiol was replaced with thiophenol. One reaction step is omitted as the thiophenol group may be substituted with 1,2-diaminoethane directly without prior oxidation of the sulfide to the sulfone. However, thiophenol is highly malodorous and quite toxic.

The use of boronate ester protection in the synthesis of caspofungin is described in US 5,936,062. Prior to borane reduction of the primary amide, the two vicinal diol systems are protected as the phenylboronate esters. Acidic work-up of the reaction mixture then releases reduced pneumocandin B₀ in 61% yield. The patent claims the bis(phenylboronate) derivative of pneumocandin B₀.

When pneumocandin B₀ is reacted with phenylboronic acid prior to treatment with thiophenol under acid catalysis, certain process impurities may be minimised, cf. US 7,214,768. Epimerisation of the benzylic position, as well as substitution of the benzylic hydroxy function, is suppressed when the benzylic alcohol is derivatised as a boronate ester.

Furthermore, in WO 2007/057141, yet another method for synthesising caspofungin is disclosed. The process relies on the two-step reduction of the primary amide to the amine via the corresponding nitrile and includes new intermediates.

Leonard and co-workers at Merck Research Laboratories provide a detailed description of the development of the caspofungin synthesis, Leonard et al., J. Org. Chem. 2007, 72, 2335-2343. The phenylboronate ester protection is included in the reduction of the primary amide, as well as during the insertion of thiophenol under acid catalysis. The overall yield of caspofungin from pneumocandin B₀ is reported to be 45%. The process consists of three chemical reaction steps and two chromatographic purifications. The reduction of the primary amide was performed at -10 to 0 °C.

As substitution of the hemiaminal hydroxy function directly with 1,2-diaminoethane has been found difficult (Leonard *et al.*), a two-step sequence has been necessary in the introduction of the ethylenediamine side-chain. According to the prevailing method disclosed in the prior art, sulphur nucleophiles such as thiophenol have been used as helping groups since they are readily substituted for the hemiaminal hydroxy function and may be expelled by a second nucleophile (*i.e.* 1,2-diaminoethane) directly or after oxidation of the thiophenol. In particular, thiophenol has been successful as a helping group in the caspofungin synthesis. However, thiophenol is highly malodorous and toxic, and using it in production scale demands special equipment.

A highly efficient method of substituting the hemiaminal hydroxy function with 1,2-diaminoethane is disclosed in US 8,048,853 B2. In this patent it was found that certain nitrogen nucleophiles, such as tetrazole and pyridine may replace thiophenol as the helping group in the introduction of the caspofungin aminal side-chain.

A main step in the caspofungin synthesis is the reduction of the amide to a primary amine. Known synthetic methods for reducing pneumocandin B₀ involving in particular the use of cyanuric chloride for the transformation from primary amide to the nitrile have several disadvantages. First of all, a very low temperature is required (-30°C) due to the high reactivity of the reagent and the instability of pneumocandin B₀. Further, this method requires a very small and specific amount of water to ensure that the reagent is not too reactive. If the temperature is too high large amount of the unwanted benzylic isomer is formed and the yield is lowered. The cyanuric chloride is lethal by inhalation and the solvent used is a high quality *N,N*-dimethylformamide solvent, which is both expensive and a class 2 reagent.

The current methods of manufacturing caspofungin involve many steps of synthesis and purification, and a substantial amount of material is lost during the process. In particular, the current synthetic steps used to reduce the primary amide leaves room for improvement both in terms of increased product yield and reduced amounts of biproducts. Thus, the primary goal of the present invention is to provide an improved method of manufacturing caspofungin, in particular a method wherein the primary amide is reduced in an improved manner.

### Summary of the invention

The present invention is defined by the attached claims and in the following:
In a first aspect, the present invention provides a process for the production of a compound of formula I 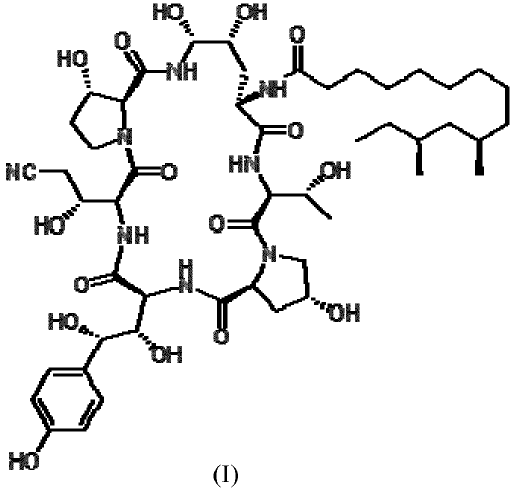 comprising the step of dehydrating a compound of formula II 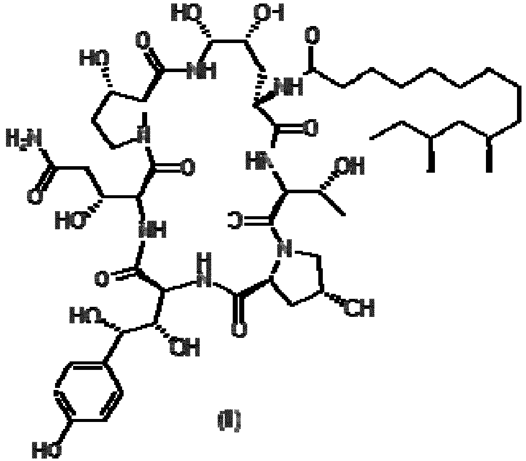 wherein the dehydration step comprises dissolving the compound of formula II and a Pd(II) catalyst in a solvent mixture comprising acetonitrile and water.

The dehydration step provides a final reaction mixture comprising the compound of formula I.

The term "final reaction mixture" is intended to mean the mixture comprising the compound of formula I and the Pd(II) catalyst obtained when the dehydration step is complete.

The term "dissolving" is also intended to cover situations wherein some of the compound of formula II is only suspended in the solvent mixture.

The ratio of acetonitrile (ACN) and water (H₂O), ACN:H₂O, may preferably be in the range of about 5:1 to about 1:5, about 4:1 to about 1:4, about 3:1 to about 1:3, about 2:1 to about 1:2 and even more preferred in the range of about 1:1 to about1:2.

In the process for the production of a compound of formula I, an initial concentration of the compound of formula II in the solvent mixture may be up to 1.0 M, up to 0.5 M, and preferably in the range of about 0.005 to about 0.5 M, about 0.01 to about 0.1 M or about 0.015 to about 0.08 M.

The process for the production of a compound of formula I may comprise a step of removing the Pd(II) catalyst from the final reaction mixture. The Pd(II) catalyst may be removed by any suitable method in the prior art, such as passing the reaction mixture through a suitable column or filter (e.g. filtration through a Celite plug), optionally after adding a Pd-scavenger (e.g. a solid support metal scavenger such as Quadasil MP).

The process for the production of a compound of formula I may comprise a step of removing the solvent mixture, or at least the acetonitrile, from the final reaction mixture. The step of removing the solvent mixture, or at least the acetonitrile, may be performed before, after, or simultaneously with, the step of removing the Pd(II) catalyst from the final reaction mixture. Preferably the step of removing the solvent mixture is performed after the step of removing the Pd(II) catalyst from the final reaction mixture. The solvent mixture may be removed by any suitable method of the prior art, such as evaporation, freeze drying, spray drying, precipitation.

The process for the production of a compound of formula I may comprise a step of removing the acetonitrile (ACN) from the final reaction mixture. The acetonitrile may be removed by any suitable method of the prior art, such as evaporation, freeze drying, spray drying, precipitation and chromatography (solid phase extraction, SPE).

The solvent mixture may comprise a polar co-solvent such as methanol, ethanol, propanol, butanol, tetrahydrofuran or dimethylformamide. The co-solvent may be present in an amount of about 0.1 to about 20 % v/v.

The dehydration step may include stirring the reaction mixture, the reaction mixture comprising the compound of formula II, the Pd(II) catalyst and the solvent mixture.

The Pd(II) catalyst may be any palladium (II) salt, such as PdCl₂, PdBr₂, Pdl₂, Pd(acac)₂ and Pd(OAc)₂ soluble in the chosen solvent mixture. Included are also complexes comprising Pd(II) salts and weakly coordinating ligands such as PdCl₂(CH₃CN)₂ that is soluble in the chosen solvent system. The amount of Pd(II) catalyst is preferably in the range of about 0.5 to about 20 mol% (relative the compound of formula II), about 0.5to about 10 mol% or about 0.5-5 mol%.

The dehydration step is preferably performed at a temperature in the range of about 0 to about 80 °C, about 10 to about 60 °C, about 10 to about 50 °C, about 0 to about 35 °C, about10 to about 35 °C or preferably about 20 to about 35 °C.

The reaction time is preferably from 1h to 96h or preferably 12h to 24h.

In a second aspect, the present invention provides a process for the production of a compound of formula III, or a pharmaceutically acceptable salt thereof 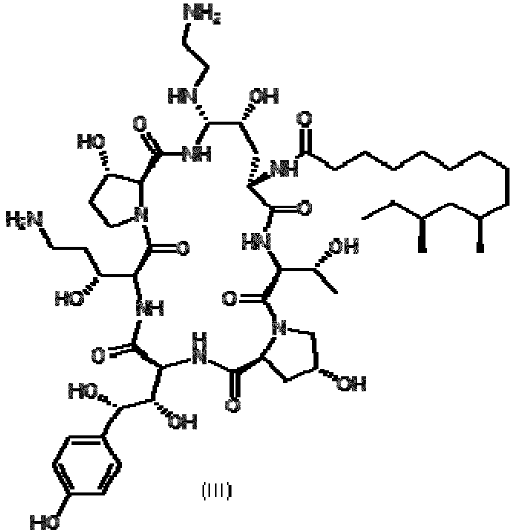 comprising the step of dehydrating a compound of formula II 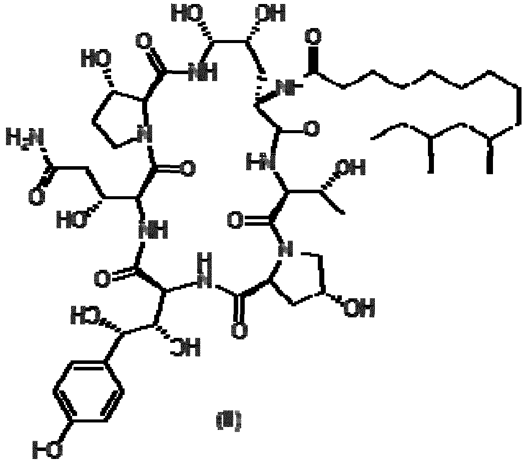 to obtain a compound of formula I 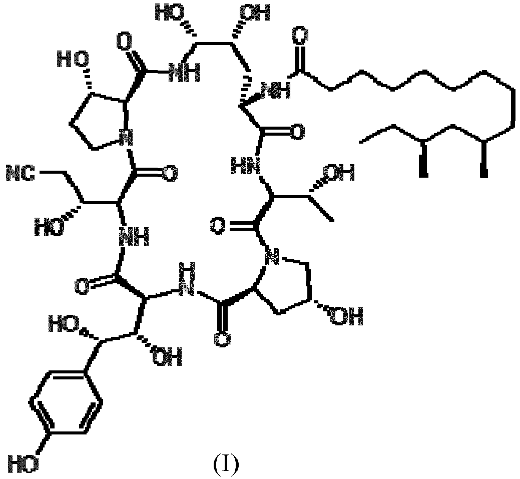 wherein the dehydration step comprises dissolving the compound of formula II and a Pd(II) catalyst in a solvent mixture comprising acetonitrile and water.

The dehydration step may comprise any of the technical features described in connection with the dehydration step in the first aspect of the invention.

The dehydration step may comprise a step of evaporating the solvent mixture.

In an embodiment of the second aspect, the process comprises a step of reducing the compound of formula II to obtain a compound of formula IV

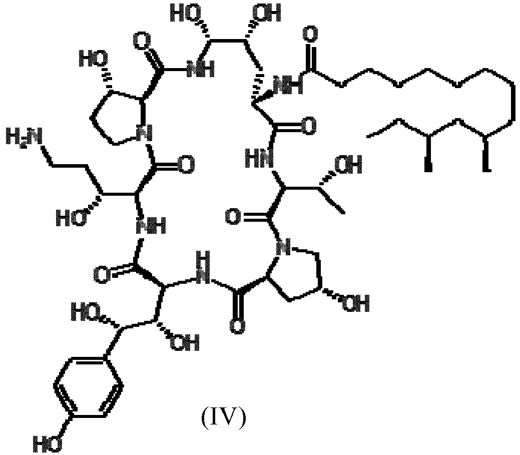

The reducing step may comprise reacting the compound of formula II, dissolved in a suitable solvent, with hydrogen or a hydrogen source in the presence of a suitable hydrogenation catalyst (i.e. a hydrogenation step), or reacting the compound of formula II with a suitable reducing agent, such as BH₃*THF.

Methods for hydrogenating the compound of formula II, in addition to suitable solvents and catalysts for the hydrogenation step, are well known in the prior art and are disclosed in for instance US 8,048,853 B2, Leonard et al., J. Org. Chem. 2007, 72, 2335-2343 and Journet et al., J. Org. Chem. 1999, 64, 2411-2417; the contents of which are incorporated by reference. The hydrogenation step reducing the nitrile moiety to an amine may be performed by any of the known prior art methods in a manner well known to the skilled person. A method of reducing the compound of formula II by use of a borane is also disclosed in Leonard et al., J. Org. Chem. 2007, 72, 2335-2343. However, the use of borane to reduce the nitrile may require additional steps of adding various protecting groups.

In an embodiment of the second aspect, the process further comprises a substitution reaction at the hemiaminal moiety of the compound of formula IV to obtain the compound of formula III

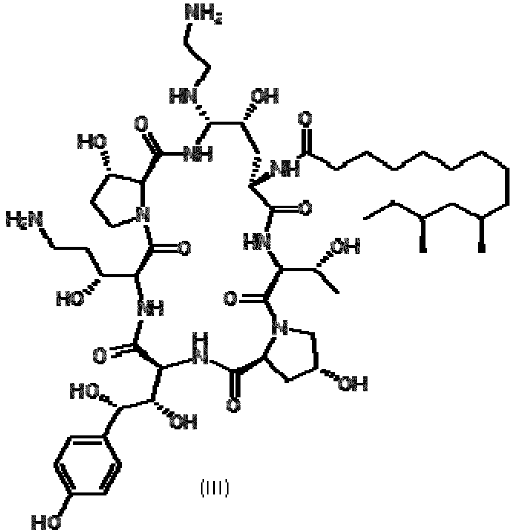

Substitution reactions at the hemiaminal moiety of the compound of formula IV to obtain the compound of formula III are well known in the prior art and are preferably performed by a two-step sequence. In other words, the substitution reaction may be performed in any manner as disclosed in the prior art. In particular, the substitution reaction may be performed as disclosed in US 8,048,853 B2, Leonard et al., J. Org. Chem. 2007, 72, 2335-2343 and US 5,378,804, the contents of which are incorporated by reference. A particularly preferred method of obtaining the compound of formula III by a two-step sequence (i.e. a double substitution reaction at the hemiaminal moiety) is the one disclosed in US 8,048,853 B2.

In an embodiment of the second aspect, the reduction or hydrogenation step (i.e. the reduction of the nitrile moiety to an amine) may alternatively be performed on derivatives of the compound of formula I, wherein the hemiaminal hydroxy group has been substituted by a leaving group, such as thiophenol or tetrazole. Such derivatives may also feature protecting groups at the benzylic hydroxy group of the compound of formula I.

### Detailed description of the invention

The present invention will now be described in more detail with reference to figures and examples. The following description and examples intend to illustrate the present invention and should in no way be considered limiting. Furthermore, the skilled person will acknowledge that various modifications may be introduced without departing from the scope of the invention. Accordingly, other embodiments of the present invention which are within the abilities of the skilled person are to be understood to be within the scope of the enclosed claims.

Based on the prior art as disclosed in the background section it is clear that the current synthetic methods used to reduce the primary amide of pneumocandin B₀ by way of the corresponding nitrile leaves room for improvement. In particular the synthetic methods of the prior art used to dehydrate the primary amide to the corresponding nitrile leaves room for improvement.

Various methods for dehydrating primary amides to nitriles are known in the prior art. For a review of known methods, see International Journal of Chemistry and Applications, Vol. 4, No. 4 (2012), pp. 295-304.

Maffioli and co-workers reported a mild and reversible dehydration of primary amides with palladium chloride in aqueous acetonitrile (Maffioli et al., Org Lett. 2005, Vol 7, 23, 5237-5239). This group reported the transformation from a primary amide to a nitrile in simple aromatic systems and only one aliphatic system with moderate to good yields. PdCl₂ generated a reaction solution with pH in the range 2.8-3.5, whereas Pd(OAc)₂ generated a solution with pH in the range 4.7-5 and the reaction temperatures were in the range of ambient temp to 50 °C. Due to the low pH of the reaction solution and the elevated temperatures of the reaction it was considered highly unlikely that this method would work in dehydrating the primary amide of pneumocandin B₀. The uncertainty was based on the known instability of pneumocandin B₀ towards acidic conditions and relatively high temperatures, as well as the presence of functional groups that may not tolerate the Pd-catalysis, in particular the hemiaminal group.

Nevertheless, pneumocandin B₀ (also termed compound II) was subjected to conditions similar to the ones disclosed by Maffioli et al, see scheme 1.

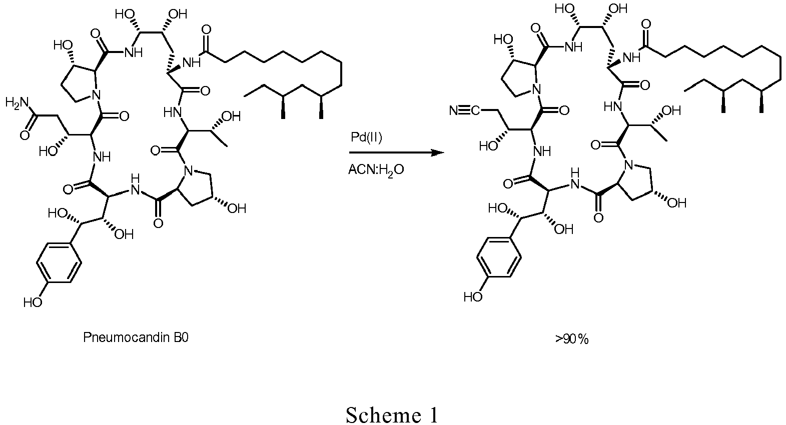

The desired dehydrated compound was surprisingly obtained in excellent yields and purity. using pneumocandin B₀ as the starting material, even at low Pd(II) catalyst loadings of 1%. Solvent mixtures of water and acetonitrile (ACN) provided a substantially quantitative dehydration/conversion of pneumocandin B₀ to the nitrile of formula I. The nitrile of formula I is usually not further purified and/or isolated since the reaction provides the nitrile in a purity sufficient to be used in subsequent synthetic process steps. However, as discussed below, the pneumocandin B₀ starting material has a purity of 96% and the substantially full conversion obtained by the inventive process is expected to commonly provide an isolated yield of above 90%.

The dehydration reaction of pneumocandin B₀ is very robust despite the well-known stability issues discussed above. The reaction tolerated both various types of Pd(II)-catalysts and loading, reaction times, pH and temperatures.

The loading of the catalyst could be varied from about 0.5 mol% to about 50 mol% and still produce the nitrile in more than 90% purity. The degradation of pneumocandin B₀ to unwanted biproducts, such as benzylic isomers was very slow compared to the degradation observed in the prior art dehydration process using cyanuric chloride. In the present dehydration reaction only about 0.2% of the biproduct formed by benzylic isomerization was detected. The wanted nitrile product could even be obtained by a substantially quantitative dehydration/conversion of pneumocandin B₀ in 90% purity (by HPLC) after 4 days of stirring at room temperature in ACN/water.

Using a solvent system of ACN:H₂O 1:1 resulted in the wanted nitrile in 94% purity (by HPLC). It is noted that the starting compound has a purity of 96% by HPLC and the conversion of the reaction is thus substantially quantitative. Solvent systems using ACN:H₂O in the range of 5:1 and 1:5 also gave the desired nitrile in more than 90% purity.

In the prior art it is stated that pneumocandin B₀ is unstable outside the pH-range of 5 to 8. However, the dehydration worked very well using PdCl₂ at both pH 3.8 and 4.8 in the ACN:H₂O 1:1 at 25 °C. Both reactions provided a substantially full conversion to the wanted nitrile having a 93% purity by HPLC.

The reaction was run at temperatures in the range of 15 °C to 50 °C. As expected, the speed of the reaction increases with increasing reaction temperatures. At lower reaction temperatures, such as 15 °C, the reaction rate is significantly slower and the reaction mixture is initially a thick suspension which becomes clear when the level of nitrile is >80%. At higher reaction temperatures, such as 50 °C, the reaction rate is very fast and the purity is somewhat lower due to increased formation of impurities. At 50 °C, the HPLC purity of the nitrile was 90% after 19 h, however an impurity of 3% was formed close to the main peak which increases over time.

During workup of the dehydration reaction, the Pd-catalyst is preferably removed before the acetonitrile is removed from the reaction mixture. This is to avoid any potential formation of pneumocandin B₀ due to the reversibility of the reaction. As discussed below, this has been observed to happen when the Pd-catalyst is present when the solvent is removed from the reaction mixture. The Pd-catalyst may be removed by known prior art methods, such as passing the reaction mixture through a suitable column, optionally after adding a suitable metal scavenger.

Caspofungin could subsequently be obtained by known methods for reduction of the nitrile group and introduction of the ethylenediamine group at the hemiaminal moiety.

### Examples

The present invention will now be described in more detail with reference to examples, which are not to be contemplated as restrictive or limiting to the scope of the present invention and the enclosed claims.

HPLC analyses were performed with a Waters Symmetry C18 column, 250 x 4.6 mm, 100 Å, 5 µm; Column temperature: 45 °C; Mobile phase: Solution A: 0.1% v/v aqueous perchloric acid, Solution B: acetonitrile, gradient elution from 67/33 A:B to 35/65 A:B; Flow: 1.5 mL/min; Detection: 205 nm; Integrator setting: Peak area%; Solvent solution: acetonitrile/water 1:1. Mass spectra were obtained with electrospray ionisation, and the instrument was run in a positive mode. Analytes have been detected in the protonated form.

### Example 1

Pneumocandin Bo (50 g, 47.0 mmol) was added to a solution of acetonitrile (500 mL) and water (500 mL) and stirred until a clear solution was obtained. PdCl₂ (0.416 g, 2.35 mmol, 5%) was added to the clear solution. The pale yellow solution was stirred at 35 °C temperature with N₂ (g) for 22 h. The reaction mixture was quenched by adding Quadrasil MP (4 g) and stirred for 1 h before it was filtered. The crude mixture was loaded to an SPE column (Amberlite XAD1600N) and eluted with 95% isopropanol. The solution was used directly in a subsequent hydrogenation step. HPLC purity of the nitrile: 94%.

### Example 2

Pneumocandin Bo (1 g, 0.96 mmol) was added to a solution of acetonitrile (15 mL) and water (15 mL) and stirred until a clear solution was obtained (ca. 5 min). PdCl₂ (0.0017 g, 0.009 mmol, 1%) was added to the clear solution. The pale yellow solution was stirred at 35 °C temperature with N₂ (g) for 24 h. The reaction mixture was quenched by adding Quadrasil TU (1 g) and stirred 1 h before it was filtered. The crude mixture was loaded to an SPE column (Amberlite XAD1600N) and eluted with 95% isopropanol. HPLC purity of the nitrile: 92.6%.

### Example 3

Pneumocandin B₀ (1 g, 0.96 mmol) was added to a solution of acetonitrile (30 mL) and water (30 mL) and stirred until a clear solution was obtained (ca 5 min). Pd(OAc)₂ (0.021 g, 0.094 mmol, 5%) was added to the clear solution. The pale yellow solution was stirred at 25 °C temperature with N₂ (g) for 28 h. The reaction mixture was quenched by adding Quadrasil TU (0.1 g) and stirred 1 h before it was filtered. The crude mixture was loaded to an SPE column (Amberlite XAD1600N) and eluted with 95% isopropanol. HPLC purity of the nitrile: 93.0%.

Example 4 Pneumocandin B₀ (1 g, 0.96 mmol) was added into a solution of acetonitrile (30 mL) and water (30 mL) and stirred until a clear solution was obtained (ca 5 min). Pd(OAc)₂ (0.021 g, 0.094 mmol, 5%) was added to the clear solution. The pale yellow solution was stirred at 25 °C temperature with N₂ (g) for 96 h. The reaction mixture was quenched by adding Quadrasil TU (0.1 g) and stirred 1 h before it was filtered. The crude mixture was loaded to an SPE column (Amberlite XAD1600N) and eluted with 95% isopropanol. HPLC purity of the nitrile: 90.5%.

Example 5 Pneumocandin B₀ (1 g, 0.94 mmol) was added to a solution of acetonitrile (10 mL) and water (10 mL) and stirred until a clear solution was obtained (ca 5 min). PdCl₂ (0.008 g, 0.047 mmol, 5%) was added to the clear solution. The pale yellow solution was stirred at 50 °C temperature with N₂ (g) for 19 h. The reaction mixture was analyzed by HPLC. HPLC purity of the nitrile: 89%.

### Example 6

Pneumocandin B₀ (1 g, 0.94 mmol) was added to a solution of acetonitrile (10 mL) and water (10 mL) and stirred at 15 °C (ca 15 min). PdCl₂ (0.008 g, 0.047 mmol, 5%) was added to the white suspension. The white suspension was stirred at 15 °C temperature with N₂ (g) for 43 h. The reaction mixture was analyzed by HPLC. HPLC purity of the nitrile: 85%.

### Example 7

Pneumocandin B0 (8 g) was added to a solution of acetonitrile (80 mL) and water (80 mL) and PdCl₂(0.067 g) was added to the clear solution. The reaction mixture was stirred for 19 h at 35 °C. Quadrasil MP (1.6 g, 1 w/v %) was added. The metal scavenger was filtered off and the clear solution was diluted with water (160 ml) and freeze dried. Isolated yield 7.71g (98%), HPLC purity 93.1%.

### Example 8

Pneumocandin B0 (1 g) was added to a solution of acetonitrile (10 mL) and water (10 mL), and PdCl₂(0.008 g) was added to the clear solution. The reaction mixture was stirred for 18 h at 35 °C. HPLC purity was 92.6% before workup. The solvents were removed by evaporation, during which the purity of the nitrile decreased to 89.6 % and the B0 increased to 2.7 %.

In examples 1-4 and 7, the reaction was quenched by adding a metal scavenger (Quadrasil TU or Quadrasil MP) to avoid potential formation of the starting compound due to the reversibility of the reaction, as shown in example 8.

## Claims

1. A process for the production of a compound of formula I 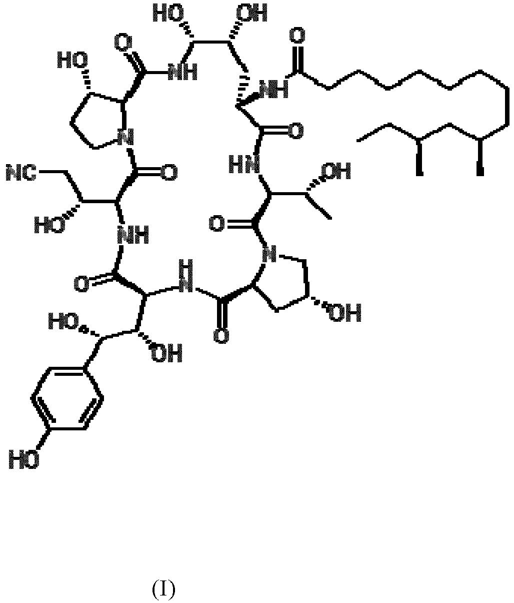 comprising the step of dehydrating a compound of formula II 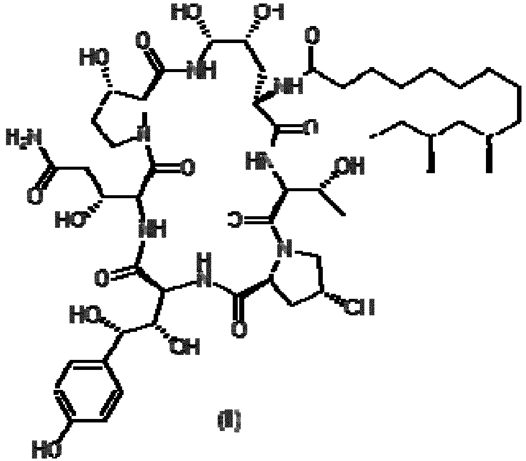 wherein the dehydration step comprises dissolving the compound of formula II and a Pd(II) catalyst in a solvent mixture comprising acetonitrile and water.

2. A process according to claim 1, wherein the ratio of acetonitrile and water is within the range of about 5:1 to about 1:5, about 4:1 to about 1:4, about 3:1 to about 1:3, about 2:1 to about 1:2 or about 1:1 to about 1:2.

3. A process according to claim 1 or 2, wherein the dehydration step is performed at a temperature within the range of about 0 to about 80 °C, about 10 to about 60 °C, about 10 to about 50 °C, about 0 to about 35 °C, about 10 to about 35 °C or about 20 to about 35 °C.

4. A process according to any of the preceding claims, comprising a step of removing the Pd(II) catalyst from a final reaction mixture comprising the compound of formula I and the solvent mixture.

5. A process according to any of the preceding claims, comprising a step of removing at least the acetonitrile from a final reaction mixture comprising the compound of formula I and the solvent mixture.

6. A process according to claims 4 and 5, wherein the step of removing the Pd(II) catalyst is performed prior to, or simultaneously with, the step of removing at least the acetonitrile.

7. A process for the production of a compound of formula III, or a pharmaceutically acceptable salt thereof 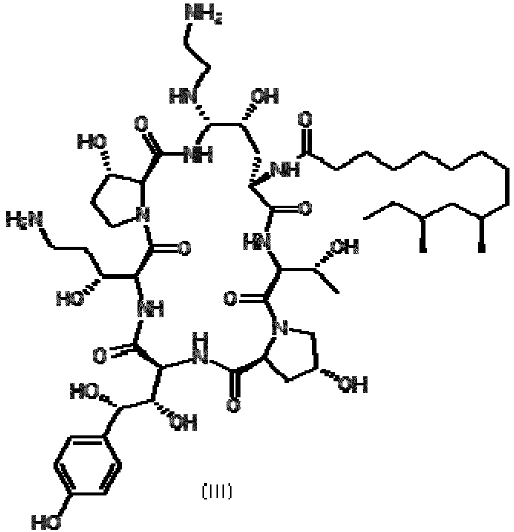 comprising the step of dehydrating a compound of formula II 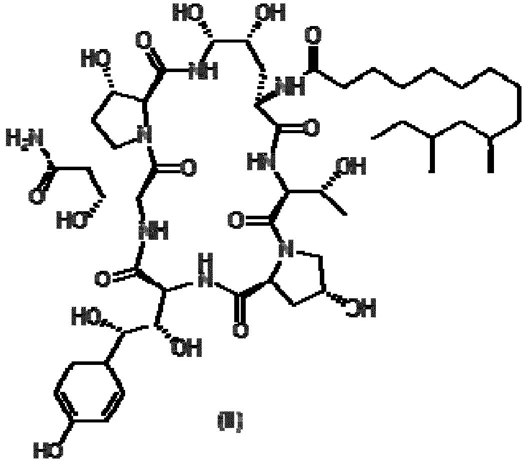 to obtain a compound of formula I 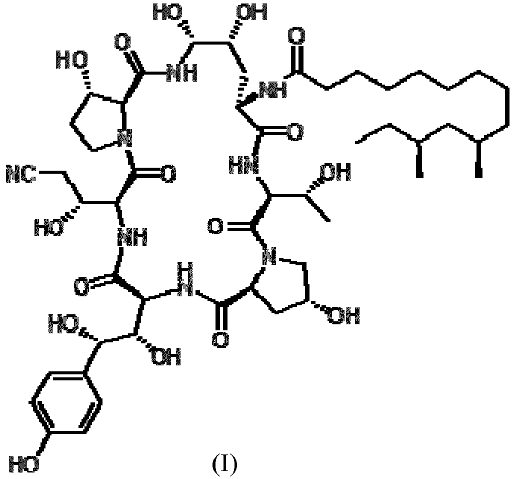 wherein the dehydration step comprises dissolving the compound of formula II and a Pd(II) catalyst in a solvent mixture comprising acetonitrile and water.

8. The process according to claim 7, further comprising a step of reducing the compound of formula II to obtain a compound of formula IV

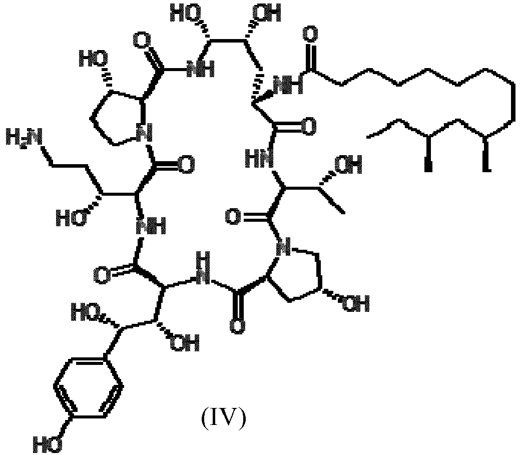

9. The process according to claim 7 and 8, further comprising a substitution reaction at the hemiaminal moiety of the compound of formula IV to obtain the compound of formula III
